# EUROPEAN PATENT APPLICATION

(11) **EP 3 949 947 A1**
(43) Date of publication of application: **09.02.2022**
(21) Application number: 20784670.0
(22) Date of filing: 03.04.2020
(51) Int. Cl.: A61K 8/96, A61K 8/27, A61K 8/29, A61K 8/49, A61Q 1/00, A61Q 19/00

(54) **POWDER COSMETIC CONTAINING ULTRAVIOLET WAVELENGTH-CONVERTING SUBSTANCE, AND METHOD FOR PRODUCING SAME**

(30) Priority: 05.04.2019 JP 2019072750
(71) Applicant: Shiseido Company, Ltd., Chuo-ku Tokyo 104-0061 (JP)
(72) Inventor: YOSHIDA, Rina, Tokyo 104-0061 (JP); MIYAZAWA, Kazuyuki, Tokyo 104-0061 (JP); KANEMARU, Tetsuya, Tokyo 104-0061 (JP); GILLET, Renaud, Tokyo 104-0061 (JP); MCCARTHY, Bianca, Tokyo 104-0061 (JP)
(74) Representative: Cabinet Beau de Loménie
(86) International application number: PCT/JP2020/015423
(87) International publication number: WO 2020/204192

(57) **Abstract**

There is provided a novel production method of a powder cosmetic containing a UV wavelength conversion substance including a slurry step, and a powder cosmetic produced by this method.

## Description

### FIELD

The present invention relates to a powder cosmetic having a cell activation action and comprising a UV wavelength conversion substance and a production method therefor.

### BACKGROUND

Ultraviolet is considered to generate free radicals *in vivo* and thereby cause oxidation of sebum and damage to cellular DNA. Examples of the damage caused by ultraviolet to skin include adverse effects such as skin cancer, photoaging, spots, wrinkles, and inflammation. These are undesirable from a health and beauty perspective. Although ultraviolet has been used for the purpose of sterilization, it is currently focused on protection from rather than active use of ultraviolet in consideration of the balance with harmful effects caused by ultraviolet.

Thus, many measures have been taken to protect skin from ultraviolet. Examples thereof include use of sunscreens, indoor activities avoiding sunlight, and use of UV-cut hats or clothing, and ultraviolet cut films.

For example, PTL 12 describes a foundation containing calcium manganese aluminate in Example 3, but does not describe a production method including a slurry step or a UV wavelength conversion substance for bringing about a cell activation effect.

### [CITATION LIST]

### [PATENT LITERATURE]

[PTL 1] Japanese Registered Patent Publication No. 6424656 [PTL 2] Japanese Registered Patent Publication No. 6361416
[PTL 3] WO 2018/004006
[PTL 4] Japanese Unexamined Patent Publication (Kokai) No. 2018-131422
[PTL 5] Japanese Unexamined Patent Publication (Kokai) No. 5-117127
[PTL 6] Japanese Registered Patent Publication No. 4048420
[PTL 7] Japanese Registered Patent Publication No. 4677250
[PTL 8] Japanese Registered Patent Publication No. 3303942
[PTL 9] Japanese Unexamined Patent Publication (Kokai) No. 2017-88719
[PTL 10] WO 2018/117117
[PTL 11] Japanese Unexamined Patent Publication (Kokai) No. 2015-120682
[PTL 12] WO 2017/142057

### SUMMARY

### [TECHNICAL PROBLEM]

An object of the present invention is to provide a novel powder cosmetic having a cell activation action utilizing ultraviolet and a production method therefor.

### [SOLUTION TO PROBLEM]

The present inventors have conducted intensive studies so that ultraviolet can be effectively used for skin. As a result, the present inventors have conceived of novel powder cosmetic having an excellent cell activation action and containing a UV wavelength conversion substance and a production method therefor.

The present application provides the following inventions.
(1) A production method of a powder cosmetic containing a UV wavelength conversion substance, comprising a slurry step.
(2) The production method according to (1), which is a wet production process.
(3) The production method according to (1), which is a wet-mixing/droplet-drying process.
(4) The production method according to any one of (1) to (3), wherein the UV wavelength conversion substance comprises one or more UV wavelength conversion substances selected from: phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium.
(5) The production method according to any one of (1) to (4), wherein the content of the UV wavelength conversion substance is 0.01 to 20% by weight relative to the total of the powder cosmetic.
(6) A production method of a powder cosmetic containing one or more phosphor substances selected from: phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium, the method comprising a slurry step.
(7) The production method according to (6), wherein the content of the phosphor substance is 0.01 to 20% by weight relative to the total of the powder cosmetic.
(8) The production method according to any one of (1) to (7), wherein the powder cosmetic is a foundation, eyeshadow, cheek color, body powder, perfume powder, baby powder, pressed powder, deodorant powder, loose powder, or face powder.
(9) The production method according to any one of (1) to (8), wherein a fluorescence intensity increasing effect is exhibited.
(10) The production method according to any one of (1) to (9), wherein a cell activation effect is exhibited.
(11) A powder cosmetic produced by the production method according to any one of (1) to (10).
(12) A wet powder cosmetic comprising a UV wavelength conversion substance.
(13) The wet powder cosmetic according to (12), wherein the UV wavelength conversion substance comprises one or more UV wavelength conversion substances selected from:
   phycocyanin, phycoerythrocyanin, phycoerythrin, vitamin A, β-carotene, vitamin K, vitamin B1, vitamin B2, vitamin B2 derivatives, vitamin B6, vitamin B 12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104,
   meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium.
(14) The wet powder cosmetic according to (12) or (13), wherein the content of the UV wavelength conversion substance is 0.01 to 20% by weight relative to the total of the cosmetic.
(15) A wet powder cosmetic comprising one or more phosphor substances selected from:
   phycocyanin, phycoerythrocyanin, phycoerythrin, vitamin A, β-carotene, vitamin K, vitamin B1, vitamin B2, vitamin B2 derivatives, vitamin B6, vitamin B 12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium.
(16) The wet powder cosmetic according to (15), wherein the content of the phosphor substance is 0.01 to 20% by weight relative to the total of the powder cosmetic.
(17) The wet powder cosmetic according to any one of (12) to (16), which is a foundation, eyeshadow, cheek color, body powder, perfume powder, baby powder, pressed powder, deodorant powder, loose powder, or face powder.
(18) The wet powder cosmetic according to any one of (12) to (17), which exhibits a fluorescence intensity increasing effect.
(19) The wet powder cosmetic according to any one of (12) to (18), which exhibits a cell activation effect.

### [ADVANTAGEOUS EFFECTS OF INVENTION]

The UV wavelength conversion substance of the present invention is suitable for effectively utilizing ultraviolet to activate skin cells. The aspect of the powder cosmetic according to the production method of the present invention is suitable so that the UV wavelength conversion substance converts ultraviolet to visible light. Conventionally, since ultraviolet is not preferable for skin, it is a technical common knowledge in this field to take measures to avoid exposing skin to ultraviolet as much as possible. However, the present invention is based on the knowledge that conversely a UV wavelength conversion substance utilizes ultraviolet to activate cells and thereby provide a preferable action on skin, and is surprising. Thus, the cosmetic of the present invention may lead to an improvement in the quality of life, such that even those who have avoided ultraviolet as much as possible for a reason of beauty or health may feel like going out actively.

### BRIEF DESCRIPTION OF DRAWINGS

FIG. 1 is a schematic diagram of Experiments 1 and 2.
FIG. 2 shows the cell activity at UV irradiation using each ultraviolet in Experiment 1. The vertical axis indicates relative fluorescence intensity (au).
FIG. 3 shows the cell activity at UV irradiation of each intensity using C-phycocyanin in each concentration in Experiment 2 as the relative fluorescence intensity (au).
FIG. 4 is a schematic diagram of Experiment 3.
FIG. 5 shows the cell activity when irradiating cells having cell activity temporarily reduced in Experiment 3 with UV using C-phycocyanin as relative fluorescence intensity (au) (P test).

### DESCRIPTION OF EMBODIMENTS

The present invention will be described below in detail with reference to specific embodiments. However, the present invention is not limited to the following embodiments, and can be carried out in any embodiments without departing from the spirit of the present invention.

All of the patent publications, unexamined patent publications, and non-patent literature cited in the present disclosure are incorporated by reference in their entirety into the present disclosure for all purposes.

In the present disclosure, "to" when applied to numerical values refers to a range of values that fall within a range that is greater than or equal to a defined reference value and less than or equal to a defined reference value.

### Production method

The production method of the powder cosmetic of the present invention comprises a slurry step. The phrase "slurry step" as used herein refers to a step in which when producing a powder cosmetic, the composition of the cosmetic raw material is processed in the state of a slurry (a fluid in which solid components such as a powder are suspended in a liquid) in a volatile dispersion medium.

For example, a production method of a powder cosmetic in which wet mixing is performed by adding powder components and an oil content to a volatile dispersion medium to form a slurry, a container is then filled with the mixture in the state of slurry, and the solvent is removed by vacuum suction to obtain a powder solid (referred to as wet forming) is known (Japanese Examined Patent Publication (Kokoku) No. 57-60004, Japanese Examined Patent Publication (Kokoku) No. 61-54766), which is referred to as a "wet production process." The wet production process is a production method comprising a slurry step.

A production method wherein a pulverized solution obtained by pulverizing a polymer powder in a volatile dispersion medium using a medium stirring mill is obtained, the pulverized solution and a powder such as a pigment are then mixed with a wet mixer such as a disperser to obtain a slurry, and the volatile dispersion medium is then removed from the obtained slurry to obtain a dry powder, which is further pulverized with a pulverizer and then dry-molded to obtain a solid powder cosmetic (Japanese Unexamined Patent Publication (Kokai) No. 9-30926), and a production method for a powder cosmetic wherein a container is filled with a slurry obtained using a medium stirring mill in wet mixing followed by suction pressing for wet molding or the solvent is removed from the obtained slurry to obtain a dry powder, the dry powder is further crushed with a pulverizer followed by dry molding to obtain the powder cosmetic (Japanese Patent Publication No. 3608778) are known. These methods are production methods comprising a slurry step.

A production method for a powder solid cosmetic obtained by highly dispersing a slurry obtained by wet mixing using a bead mill and then drying and forming into fine particles with a flash dryer (Japanese Unexamined Patent Publication (Kokai) No. 2007-055990) is known. In the present description, this is described as a "wet-mixing/droplet-drying process." The wet-mixing/droplet-drying process is a production method comprising a slurry step.

The method for producing the powder cosmetic of the present invention is a production method comprising a slurry step, is preferably a wet production process, and is a wet-mixing/droplet-drying process.

### UV wavelength conversion substance

The powder cosmetic according to the production method of the present invention contains a UV wavelength conversion substance as an active component. The phrase "UV wavelength conversion substance" refers to a substance which converts the wavelength of ultraviolet contained in incident light to outgoing light with a wavelength longer than the wavelength of ultraviolet. The phrase "organic UV wavelength conversion substance" refers to a UV wavelength conversion substance which is an organic compound, and the phrase "inorganic UV wavelength conversion substance" refers to a UV wavelength conversion substance which is an inorganic compound.

The ultraviolet may contain UVA, UVB, or UVC. In one embodiment, the ultraviolet is light having a peak wavelength of 200 nm to 400 nm. Further, incident light, for example, sunlight, may contain ultraviolet. Furthermore, the incident light may be ultraviolet or artificially generated ultraviolet may be used.

The outgoing light emitted from the UV wavelength conversion substance has a longer wavelength than ultraviolet and has a peak wavelength of preferably 500 nm to 700 nm. The outgoing light may have one or more peaks, for example, but not limited to, at 510 nm, 520 nm, 530 nm, 540 nm, 550 nm, 560 nm, 570 nm, 580 nm, 590 nm, 600 nm, 610 nm, 620 nm, 630 nm, 640 nm, 650 nm, 660 nm, 670 nm, 680 nm, 690 nm, 700 nm, or within any range therebetween or may be red light, orange light, green light, or blue light. In one embodiment, the main wavelength exhibited by the light emitted from the UV wavelength conversion substance when excited by 200 nm to 400 nm excitation light is 500 nm to 700 nm.

Examples of the UV wavelength conversion substance include the following components: phycobiliproteins such as phycocyanin (allophycocyanin, C-phycocyanin, R-phycocyanin), phycoerythrocyanin, phycoerythrin (B-phycoerythrin, b-phycoerythrin, C-phycoerythrin, R-phycoerythrin); natural or synthetic components such as vitamin A, β-carotene, vitamin K, vitamin B1, vitamin B2, vitamin B6, vitamin B 12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, and polyphenols; colors such as Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, and meta-aminophenol; phosphors of an inorganic compound doped to exhibit a fluorescence property, such as the blue phosphor containing an amorphous silica particle, cerium, and phosphorus and/or magnesium described in Japanese Registered Patent Publication No. 6424656, the red phosphor containing a europium-activated compound of a mixed crystal of an alkaline earth metal sulfide and a gallium compound described in Japanese Registered Patent Publication No. 6361416, the zinc oxide phosphor described in WO 2018/004006, the zinc oxide phosphor described in Japanese Unexamined Patent Publication (Kokai) No. 2018-131422, the inorganic phosphor described in Japanese Unexamined Patent Publication (Kokai) No. 5-117127 (hereinafter, a phosphor derived from zinc oxide is referred to as a "zinc oxide phosphor"). In one embodiment, the inorganic phosphor is one or more phosphors selected from phosphors of zinc oxide represented by ZnO:Zn, Zn₁ + _{z} , or ZnO_{1 - x}, doped with a sulfide and/or a sulfate, such as zinc sulfide and zinc sulfate, described in WO 2018/004006; magnesium titanate phosphors of a magnesium titanate such as MgTiO₃ or Mg₂TiO₄ doped with manganese (hereinafter referred to as magnesium titanate is referred to as a "magnesium titanate phosphor"); and calcium phosphate phosphors of a calcium phosphate such as Ca(H₂PO₄)₂, CaHPO₄, or Ca₃(PO₄)₂ doped with cerium.

Further, the inorganic UV wavelength conversion substance which is an inorganic phosphor may be subjected to a surface treatment. Examples of the surface treatment include a silane compound treatment (octyltriethoxysilane), a silicone compound treatment, a fluorine-modified silicone compound treatment, a fluorine compound treatment, a higher fatty acid treatment (stearic acid, etc.), a higher alcohol treatment, a fatty acid ester treatment, a metal soap treatment, an amino acid treatment, and an alkyl phosphate treatment.

UV wavelength conversion substance may be obtained by a method of extraction from natural products such as animals, plants, and algae or an artificial method such as chemical synthesis. For example, phycobiliproteins may be prepared from blue-green algae such as *Spirulina platensis,* red algae such as *Porphyridium cruentum,* and other algae by the extraction method described in, for example, Japanese Registered Patent Publication No. 4048420, Japanese Registered Patent Publication No. 4677250, or Japanese Registered Patent Publication No. 3303942. Zinc oxide phosphors may be produced by the method described in, for example, WO 2018/004006, Japanese Unexamined Patent Publication (Kokai) No. 2018-131422, or Japanese Unexamined Patent Publication (Kokai) No. 5-117127. Magnesium titanate phosphors may be produced by the method described in Japanese Unexamined Patent Publication (Kokai) No. 2017-88719. Calcium phosphate phosphor may be produced by the method described in WO 2018/117117.

These UV wavelength conversion substances may consist of or contain these exemplified components, which may be used alone or in combination of two or more, as long as the wavelength conversion effect of the invention is not impaired. For example, to the phycobiliprotein or inorganic phosphor, there may be added another wavelength conversion substance such as vitamin B (vitamin B1, vitamin B2, vitamin B6, or vitamin B12) to aim for a synergistic effect. Note that these components are merely examples and any substance which can bring about a wavelength conversion effect can be used in the present invention. The UV wavelength conversion substance contained in the powder cosmetic according to the production method of the present invention comprises one or more UV wavelength conversion substances selected from: phycocyanin, phycoerythrocyanin, phycoerythrin, vitamin A, β-carotene, vitamin K, vitamin B1, vitamin B2, vitamin B6, vitamin B12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium.

Any derivative of vitamin B2, which is a UV wavelength conversion substance, may be used as long as the derivative is a UV wavelength conversion substance. Examples of the vitamin B2 derivatives include riboflavin acetate ester, riboflavin butyrate, riboflavin phosphate (may be a sodium or mono-diethanolamine salt), flavin mononucleotide, flavin adenine dinucleotide, riboflavin tetrabutyrate, and riboflavin tetranicotinate. Derivatives of lixoflavin, which is a stereoisomer of riboflavin, may be used.

The content of the UV wavelength conversion substance in the powder cosmetic according to the production method of the present invention is not particularly limited as long as the wavelength conversion effect of the present invention is not impaired. The content can be appropriately determined in accordance with the type of the UV wavelength conversion substance or the application of the powder cosmetic containing the UV wavelength conversion substance. The range thereof is not limited and may be, for example, 0.01 to 99.99% by weight, or 0.1% to 99.9% by weight.

As one aspect of the powder cosmetic according to the production method of the present invention, the UV wavelength conversion substance of the powder cosmetic contains a zinc oxide phosphor. In the powder cosmetic of the present invention, preferably, the content of a zinc oxide phosphor relative to the total of the powder cosmetic is 0.01% by weight or more, 0.1% by weight or more, preferably 0.5% by mass or more, or more preferably 1.0% by weight or more, and 20% by weight or less, preferably 15% by weight or less, more preferably 10% by weight or less, or even more preferably 5% by weight or less. The content relative to the total of the powder cosmetic is 0.01 to 99.99% by weight, 0.01 to 20% by weight, 0.1 to 99.9% by weight, 0.1 to 50% by weight, 0.1 to 40% by weight, 0.1 to 30% by weight, 0.1 to 20% by weight, 0.1 to 10% by weight, or 1 to 10% by weight.

As one aspect of the powder cosmetic according to the production method of the present invention, the UV wavelength conversion substance of the powder cosmetic contains a magnesium titanate phosphor. In the powder cosmetic of the present invention, preferably, the content of a magnesium titanate phosphor relative to the total of the powder cosmetic is 0.01% by weight or more, 0.1% by weight or more, preferably 0.5% by mass or more, or more preferably 1.0% by weight or more, and 20% by weight or less, preferably 15% by weight or less, more preferably 10% by weight or less, or even more preferably 5% by weight or less. The content relative to the total of the powder cosmetic is 0.01 to 99.99% by weight, 0.01 to 20% by weight, 0.1 to 99.9% by weight, 0.1 to 50% by weight, 0.1 to 40% by weight, 0.1 to 30% by weight, 0.1 to 20% by weight, 0.1 to 10% by weight, 0.5 to 10% by weight, or 1 to 10% by weight.

As one aspect of the powder cosmetic according to the production method of the present invention, the UV wavelength conversion substance of the powder cosmetic contains phycocyanin. In the powder cosmetic of the present invention, preferably, the content of a phycocyanin relative to the total of the powder cosmetic is 0.00001% by weight or more, preferably 0.0001% by mass or more, or even more preferably 0.01% by weight or more, and 20% by weight or less, preferably 15% by weight or less, more preferably 10% by weight or less, or even more preferably 5% by weight or less. The content relative to the total of the powder cosmetic is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.01 to 5% by weight, 0.05 to 5% by weight, 0.1 to 5% by weight, 0.1 to 3% by weight, 0.1 to 1% by weight, or 0.01 to 20% by weight.

As one aspect of the powder cosmetic according to the production method of the present invention, the UV wavelength conversion substance of the powder cosmetic contains vitamin B2. In the powder cosmetic of the present invention, preferably, the content of vitamin B2 relative to the total of the powder cosmetic is 0.00001% by weight or more, preferably 0.0001% by weight or more, and 20% by weight or less, preferably 15% by weight or less, more preferably 10% by weight or less, or even more preferably 5% by weight or less. The content relative to the total of the powder cosmetic is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.005 to 5% by weight, 0.01 to 5% by weight, 0.01 to 1% by weight, 0.01 to 0.5% by weight, or 0.01 to 0.1% by weight.

One aspect of the powder cosmetic according to the production method of the present invention is a powder cosmetic containing one or more phosphor substances selected from: phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium.

The content of the phosphor substance contained in the powder cosmetic according to the production method of the present invention, relative to the total of the powder cosmetic, is 0.01% by weight or more, 0.1% by weight or more, preferably 0.5% by weight or more, or more preferably 1.0% by weight or more, and 20% by weight or less, preferably 15% by weight or less, more preferably 10% by weight or less, or even more preferably 5% by weight or less. The content relative to the total of the powder cosmetic is 0.01 to 99.99% by weight, 0.01 to 20% by weight, 0.1 to 99.9% by weight, 0.1 to 50% by weight, 0.1 to 40% by weight, 0.1 to 30% by weight, 0.1 to 20% by weight, 0.1 to 10% by weight, or 1 to 10% by weight.

As one aspect of the powder cosmetic according to the production method of the present invention, the phosphor substance of the powder cosmetic contains a zinc oxide phosphor. In the powder cosmetic of the present invention, preferably, the content of a zinc oxide phosphor relative to the total of the powder cosmetic is 0.01% by weight or more, 0.1% by weight or more, preferably 0.5% by mass or more, or more preferably 1.0% by weight or more, and 20% by weight or less, preferably 15% by weight or less, more preferably 10% by weight or less, or even more preferably 5% by weight or less. The content relative to the total of the powder cosmetic is 0.01 to 99.99% by weight, 0.01 to 20% by weight, 0.1 to 99.9% by weight, 0.1 to 50% by weight, 0.1 to 40% by weight, 0.1 to 30% by weight, 0.1 to 20% by weight, 0.1 to 10% by weight, or 1 to 10% by weight.

As one aspect of the powder cosmetic according to the production method of the present invention, the phosphor substance of the powder cosmetic contains a magnesium titanate phosphor. In the powder cosmetic of the present invention, the content of a magnesium titanate phosphor relative to the total of the powder cosmetic is 0.01% by weight or more, 0.1% by weight or more, preferably 0.5% by mass or more, or more preferably 1.0% by weight or more, and 20% by weight or less, preferably 15% by weight or less, more preferably 10% by weight or less, or even more preferably 5% by weight or less. The content relative to the total of the powder cosmetic is 0.01 to 99.99% by weight, 0.01 to 20% by weight, 0.1 to 99.9% by weight, 0.1 to 50% by weight, 0.1 to 40% by weight, 0.1 to 30% by weight, 0.1 to 20% by weight, 0.1 to 10% by weight, 0.5 to 10% by weight, or 1 to 10% by weight.

As one aspect of the powder cosmetic according to the production method of the present invention, the phosphor substance of the powder cosmetic contains phycocyanin. In the powder cosmetic of the present invention, preferably, the content of a phycocyanin relative to the total of the powder cosmetic is 0.00001% by weight or more, preferably 0.0001% by mass or more, or even more preferably 0.01% by weight or more, and 20% by weight or less, preferably 15% by weight or less, more preferably 10% by weight or less, or even more preferably 5% by weight or less. The content relative to the total of the powder cosmetic is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.01 to 5% by weight, 0.05 to 5% by weight, 0.1 to 5% by weight, 0.1 to 3% by weight, 0.1 to 1% by weight, or 0.01 to 20% by weight.

As one aspect of the powder cosmetic according to the production method of the present invention, the phosphor substance of the powder cosmetic contains vitamin B2. In the powder cosmetic of the present invention, preferably, the content of vitamin B2 relative to the total of the powder cosmetic is 0.00001 % by weight or more, preferably 0.0001% by weight or more, and 20% by weight or less, preferably 15% by weight or less, more preferably 10% by weight or less, or even more preferably 5% by weight or less. The content relative to the total of the powder cosmetic is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.005 to 5% by weight, 0.01 to 5% by weight, 0.01 to 1% by weight, 0.01 to 0.5% by weight, or 0.01 to 0.1% by weight.

Examples of cell activation include, but are not limited to, promoting metabolism and turnover, improving a function, promoting proliferation, inhibiting oxidation, improving resistance to fatigue and external stimuli, and inhibiting loss of function and activity in cells, such as dermal fibroblasts and/or keratinocytes, of animals including humans. When skin cells are activated, effects such as prevention of or improvement from wrinkles, spots, skin aging, and photoaging can be expected.

The cell activation effect may be measured by measuring, for example, the viability, reducing ability, or proliferation of living cells using AlamarBlue as in the Examples. Any method can be used such as another dye assay, mitochondrial membrane potential-dependent dye assay, intracellular cytochrome c assay, elastase cleavage dye assay, ATP, ADE assay, glycolytic flux and oxygen consumption assay, collagen assay, photoaging assay, collagen glycation assay, inflammatory substances (interleukin 1α, interleukin 8, tumor necrosis factor α) assay, skin barrier function-related protein (corneodesmosin, sphingomyelin phosphodiesterase, filaggrin, involucrin, loricrin, transglutaminase 1, caspase 14) assay, angiogenesis modulator (VEGF-A, ANGPT1) assay, oxidation and/or skin stress-related protein (aromatic hydrocarbon receptor repressor, cytochrome P4501B 1, aromatic hydrocarbon receptor repressor, heme oxygenase 1) assay, or hyaluronic acid assay.

The powder cosmetic according to the production method of the present invention is suitable for performing the function of a UV wavelength conversion substance and for alleviating, or more positively improving or restoring skin damage during and after ultraviolet irradiation by activating cells. Specifically, it is suitable for collagen production or hyaluronic acid production by fibroblasts and inhibition of damage caused by photoaging and for inhibiting oxidation stress of keratinocytes, enhancing a barrier function, suppressing an inflammatory reaction, and suppressing the glycation of collagen and angiogenesis in skin.

Fluorescence intensity can be measured using a spectrofluorometer by irradiating with ultraviolet a coating film of the powder cosmetic on the surface of a substrate, as in the

Examples. The substrate may be a resin substrate composed of a polymethyl methacrylate (PMMA), nylon, or acrylic plate or an inorganic plate of glass or quartz. For example, a PMMA plate having V-shaped grooves on its surface (also refer to as "S plate": Japanese Registered Patent Publication No. 4453995) can be used. As the fluorescence intensity, a fluorescence value at a specific single wavelength or an integrated value in a specific wavelength region may be used.

### Oil content

The powder cosmetic according to the production method of the present invention contains an oil content. The phrase "oil content" refers to a hydrophobic substance that phase-separates from water, which is a component of the powder cosmetic of the present invention. The oil content that can be used in the present invention is not particularly limited and contains one or more of, for example, hydrocarbon oils, ester oils, silicone oils, liquid oils, solid fats, and higher alcohols.

Examples of the hydrocarbon oils include liquid paraffin, tetraisobutane, hydrogenated polydecene, olefin oligomer, isododecane, isohexadecane, squalane, and hydrogenated polyisobutene.

Examples of the ester oils include diisopropyl sebacate, octyl palmitate, cetyl isooctanoate (cetyl 2-ethylhexanoate), triethylhexanoin, neopentyl glycol dicaprate, triisostearin, diisostearyl malate, PPG-3 dipivalate, di-2-ethylhexyl succinate, 2-ethylhexyl 2-ethylhexanoate, polyglyceryl-6 octacaprylate, and glyceryl tri(caprylate/caprate).

Examples of the silicone oils include caprylyl methicone, dimethicone (silicone KF-96A-6T), amino-modified polysiloxane, polyether-modified polysiloxane, alkyl-modified polysiloxane, and fluorine-modified polysiloxane.

Examples of the liquid oils include avocado oil, camellia oil, macadamia nut oil, mink oil, olive oil, castor oil, jojoba oil, triglycerol, glyceryl tri(caprylate/caprate) (O.D.O), glycerol trioctanoate, and isostearic acid.

Examples of the solid fats include coconut oil, hardened coconut oil, palm oil, beef tallow, mutton tallow, Japan wax, and hardened castor oil.

Examples of the higher alcohols include isostearyl alcohol, oleyl alcohol, and butylene glycol-propylene glycol copolymer (e.g., PBG/PPG-9/1 copolymer).

The suitable blending amount of the oil content, relative to the total of the powder cosmetic, is 0.5 to 40% by mass, preferably 1 to 30% by mass, or particularly preferably 5 to 25% by mass.

### Powder

The powder cosmetic according to the production method of the present invention comprises a powder. Examples of the powder include spherical powders, functional powders, pigments, extender pigments, and pearl agents.

The spherical powder contained in the powder cosmetic according to the production method of the present invention is not particularly limited as long as it is a spherical powder that can be blended in a conventional cosmetic. Examples thereof include spherical resin powders such as polyethylene powder, polypropylene powder, polymethyl methacrylate powder, nylon powder, polytetrafluoroethylene powder, silicone powder (for example, (vinyl dimethicone/methicone silsesquioxane) crosspolymers), silicone rubber powder, silk powder, urethane powder, cellulose powder, and ethylene polyfluoride, as well as fine particulate zinc oxide.

The spherical powder may be surface-treated. Examples of surface treatments include silicone compound treatment, fluorine-modified silicone compound treatment, fluorine compound treatment, higher fatty acid treatment, higher alcohol treatment, fatty acid ester treatment, metal soap treatment, amino acid treatment, and alkyl phosphate treatment.

Examples of the functional powder which can be contained in the powder cosmetic according to the production method of the present invention include powders having functions such as rough skin improvement, whitening, and moisturizing. An example of a functional powder is barium sulfate.

The pigment which can be contained in the powder cosmetic according to the production method of the present invention is not particularly limited as long as it is a pigment that can be blended in a conventional cosmetic. Examples thereof include inorganic white pigments (for example, titanium dioxide, pigment grade titanium oxide (CR-50)); inorganic red pigments (for example, red iron oxide (colcothar), iron titanate); inorganic brown pigments (for example, γ-iron oxide); inorganic yellow pigments (for example, yellow iron oxide, loess); inorganic black pigments (for example, black iron oxide, lower titanium oxide); inorganic purple pigments (for example, mango violet, cobalt violet); inorganic green pigments (for example, chromium oxide, chromium hydroxide, cobalt titanate); inorganic blue pigments (for example, ultramarine, navy blue); pearl pigments (for example, pigments using bismuth oxychloride, fish scale foil, mica titanium, iron oxide-coated mica titanium, lower-order titanium oxide-coated mica titanium, photochromic mica titanium, in place of mica as a substrate, talc, glass, synthetic fluoro phlogopite, silica, and bismuth oxychloride, and pigments coated with a coating material, in addition to titanium oxide, low-order titanium oxide, colored titanium oxide, iron oxide, alumina, silica, zirconia, cobalt oxide, and aluminum, as functional pearl pigments, a pearl pigment whose surface is coated with resin particles (Japanese Unexamined Patent Publication (Kokai) No. 11-92688), a pearl pigment whose surface is coated with aluminum hydroxide particles (Japanese Unexamined Patent Publication (Kokai) No. 2002-146238), a pearl pigment whose surface is coated with zinc oxide particles (Japanese Unexamined Patent Publication (Kokai) No. 2003-261421), a pearl pigment whose surface is coated with barium sulfate particles (Japanese Unexamined Patent Publication (Kokai) No. 2003-61229); metal powder pigments (for example, aluminum powder, copper powder); and organic pigments such as zirconium, barium or aluminum lake (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Red No. 3, Red No. 106, Red No. 230, Red No. 505, Yellow No. 202, Yellow No. 203, and Green No. 3).

The pearl agent which can be contained in the powder cosmetic according to the production method of the present invention is a plate-like or spherical powder having an interference color, pearly luster, or metallic luster, and is a general-purpose powder in the fields of paints and cosmetic materials. When the surface of a solid cosmetic is decorated with a glossy powder, the pattern or letters obtained by decoration exhibits emphasized brightness and excellent aesthetic sensation can be given.

Examples of pearl agents include titanated mica, iron oxide-coated titanated mica, carmine-coated titanated mica, titanated mica coated with carmine and iron blue, titanated mica treated with iron oxide and carmine, titanated mica treated with iron blue, titanated mica treated with iron oxide and iron blue, titanated mica treated with chromium oxide, titanated mica treated with black titanium oxide, aluminum powder coated with acrylic resin, aluminum powder coated with silica, mica coated with titanium oxide, bismuth oxychloride coated with titanium oxide, talc coated with titanium oxide, mica coated with colored titanium oxide, synthetic mica coated with titanium oxide, silica coated with titanium oxide, alumina coated with titanium oxide, glass flakes coated with titanium oxide, polyethylene terephthalate-polymethyl methacrylate laminated film powder, bismuth oxychloride, and fish scale foil.

Examples of the commercially available products include products manufactured by Merck KGaA, such as Timiron Splendid Gold^{™}, Timiron Splendid Red^{™}, Timiron Splendid Blue^{™}, Timiron Splendid Green^{™}, Timiron Super Red^{™}, Timiron Super Blue^{™},Timiron Super Green^{™}, Timiron Super Gold^{™}, Colorona Sienna^{™}, Colorona Passion Orange^{™}, Colorona Carmin Red^{™}, and Colorona Red Gold^{™}; products manufactured by BASF SE such as Cloisonne Blue^{™},Cloisonne Green^{™}, Cloisonne Gold^{™}, Cloisonne Rouge Flambe^{™}, Cloisonne Vivid Red^{™}, Gemtone Tan Opal^{™}, Gemtone Ruby^{™}, Timica Brilliant Gold^{™}, Timica Golden Bronze^{™}, Timica Copper^{™}, Timica Soft Luster White^{™}, Duochrome RB^{™}, Duochrome RY^{™}, Duochrome YR^{™}, Duochrome YB^{™}, Duochrome RG^{™}, Duochrome BG^{™}, Duochrome BR^{™}, Duochrome GY^{™}, Flamenco Velvet^{™}, Flamenco Satina^{™}, Flamenco Red^{™}, Flamenco Blue^{™},and Flamenco Gold^{™}; products manufactured by Engelhard Corporation, such as Timica Brilliant Gold^{™}, Timica Copper^{™}, and Reflecks^{™} series; products manufactured by Topy Industries, Ltd., such as Helius R100S^{™} and Helios R100Y^{™}; and products manufactured by Nippon Sheet Glass Co., Ltd., such as Metashine^{™} series.

Examples of pearl agents include composite powders in which the surface of spherical silicon dioxide, alumina, calcium carbonate, nylon, polyethylene, polystyrene, or polymethyl methacrylate, is coated with titanium dioxide, titanium oxide, zirconium oxide, or iron oxide, and such composite powders are described in Japanese Unexamined Patent Publication (Kokai) No. 11-236315. The pearl agent is not limited thereto, and any spherical powder exhibiting luster can be widely adopted as a component of a decorative powder composition.

The extender pigment which can be contained in the powder cosmetic according to the production method of the present invention is a powder used as a diluent for maintaining the formulation form of the cosmetic and to adjust the spreadability, adhesiveness, gloss, and color tone. Examples thereof include carmine, glass powder, silicic anhydride, aluminum silicate, magnesium silicate, magnesium aluminum silicate, mica, synthetic mica, synthetic sericite, sericite, talc (which may be elastomerically-treated according to Japanese Unexamined Patent Publication (Kokai) No. 2017-214565), kaolin, silicon carbide, synthetic phlogopite iron, and boron nitride.

The suitable blending amount of the powder, relative to the total of the powder cosmetic, is 50 to 99.9% by mass, preferably 70 to 99% by mass, or particularly preferably 80 to 95% by mass.

### UV absorber

The powder cosmetic according to the production method of the present invention may contain a UV absorber. The phrase "UV absorber" refers to a substance which absorbs ultraviolet and converts it to the energy of heat or infrared. The UV absorber that can be used in the present invention is not particularly limited as long as the function of the UV wavelength conversion substance is not directly impaired. Examples of the UV absorber include salicylic acid-based UV absorbers such as homomenthyl salicylate, ethylhexyl salicylate (octyl salicylate), homosalate, and triethanolamine salicylate;
cinnamic acid-based UV absorbers such as 2-ethylhexyl para-methoxycinnamate, glyceryl diparamethoxycinnamate mono-2-ethylhexanoate, methyl 2,5-diisopropylcinnamate, 2,4,6-tris[4-(2-ethylhexyloxycarbonyl)anilino]-1,3,5-triazine, isopentyl trimethoxycinnamate trisiloxane, an isopropyl para-methoxycinnamate-diisopropylcinnamate ester mixture, a diethanolamine p-methoxyhydrocinnamate salt, and ethylhexyl methoxycinnamate (octyl methoxycinnamate);
benzoylmethane UV absorbers such as 2-phenyl-benzimidazole-5-sulfuric acid, 4-isopropyldibenzoylmethane, and 4-tert-butyl-4' -methoxydibenzoylmethane;
octocrylene, 2-ethylhexyl dimethoxybenzylidene dioxoimidazolidine propionate, 1-(3,4-dimethoxyphenyl)-4,4-dimethyl-1,3-pentanedione, cinoxate, methyl-O-aminobenzoate, 3-(4-methylbenzylidene)camphor, octyltriazone, hexyl diethylaminohydroxybenzoyl benzoate, bis(ethylhexyloxyphenol)methoxyphenyl triazine, and bis(benzotriazolyl) tetramethylbutylphenol methylene. One or more selected therefrom can be contained in the powder cosmetic.

### UV scattering agent

The powder cosmetic according to the production method of the present invention may contain a UV scattering agent. The term "UV scattering agent" refers to a substance which can reflect/scatter ultraviolet and protect skin from ultraviolet. Examples of the material of the UV scattering agent that can be used in the present invention include titanium dioxide, zinc oxide other than the UV wavelength conversion substance or phosphor substance, iron oxide, zirconium oxide, and aluminum oxide. The UV scattering agent may be fine particles or a composite thereof, and one or more selected from these can be contained in the powder cosmetic.

The titanium dioxide and zinc oxide used as the UV scattering agent may be titanium dioxide and zinc oxide used commonly in cosmetics. Preferably, those having superior dispersibility, such as those subjected to a surface treatment by a publicly known method, as needed, specifically those subjected to a hydrophobic treatment, can be contained in the powder cosmetic.

Examples of the surface treatment include a silicone treatment with methylhydrogen polysiloxane or methylpolysiloxane; a fluorine treatment with perfluoroalkyl phosphoric acid ester or perfluoroalcohol; an amino acid treatment with N-acylglutamic acid; an alkylalkoxysilane treatment with octyltriethoxysilane or octyltrimethoxysilane; and further, a lecithin treatment; a metal soap treatment; a fatty acid treatment; and an alkyl phosphate treatment. Thereamong, zinc oxide surface-treated with silicone is preferably used.

The silicone used for surface treatment is not particularly limited. Examples thereof include methylpolysiloxane, methylphenylpolysiloxane, methylhydrogen polysiloxane, methylcyclopolysiloxane, octamethylcyclotetrasiloxane, decamethylcyclopentasiloxane, dodecamethylcyclohexasiloxane, octamethyltrisiloxane, tetradecamethylhexasiloxane, dimethylsiloxane-methyl(polyoxyethylene)siloxane-methyl(polyoxypropylene)siloxane copolymer, dimethylsiloxane-methyl(polyoxyethylene) siloxane copolymer, dimethylsiloxane-methyl(polyoxypropylene) siloxane copolymer, dimethylsiloxane-methylcetyloxysiloxane copolymer, dimethylsiloxane-methylstearoxysiloxane copolymer, and various other silicones. The silicone is preferably methylhydrogen polysiloxane or methylpolysiloxane.

### Dispersant

The powder cosmetic according to the production method of the present invention may contain a dispersant. The term "dispersant" refers to a substance which adsorbs on the surface of particles dispersed in a water phase or oil phase whereby the particles can be dispersed homogenously in a water-based or oil-based medium. The dispersant that can be used in the present invention is not particularly limited as long as the function of the UV wavelength conversion substance is not impaired. Oil-based dispersants are preferable, and examples of oil-based dispersants include nonionic surfactants, cationic surfactants, anionic surfactants, silicone-based surfactants, and fatty acids. In the present invention, it is particularly preferable to use a nonionic surfactant and/or a silicone-based surfactant and/or a fatty acid that are used in conventional cosmetics and pharmaceuticals.

Preferable examples of the dispersant which can be contained in the powder cosmetic according to the production method of the present invention include PEG-10 dimethicone, bis-butyldimethicone polyglyceryl-3, PEG-polydimethylpolysiloxane ethyldimethicone, lauryl PEG-polydimethylpolysiloxane ethyldimethicone, cetyl PEG/PPG-10/dimethicone, isostearic acid, diisostearic acid polyglyceryl-2, carboxydecyl trisiloxane, PEG-12 dimethicone, and polyoxyethylene sorbitan monostearate, and combinations of two or more thereof.

### (Optional component)

Various components can be appropriately blended in the powder cosmetic according to the production method of the present invention as long as the effect of the present invention is not impaired. Examples of the various components include additive components that can be blended generally in cosmetics, such as clay minerals (dimethyldistearylammonium hectorite), chelating agents (disodium edetate hydrate), fragrances, moisturizing agents (glycerin, dipropylene glycol), preservatives, oil phase solidifying agents (sucrose tetrastearate triacetate, dextrin palmitate, palmitic acid, (behenic acid/eicosane diacid)glyceryl, N-lauroyl L-glutamic acid dibutylamide, polyamide-8), anionic surfactants, cationic surfactants, amphoteric surfactants, nonionic surfactants, water-soluble polymers, silicone-modified polysaccharides, other film-forming agents, metal ion sequestering agents, lower alcohols, polyhydric alcohols, various extracts, sugars, amino acids, organic amines, polymer emulsions, pH adjusters, skin nutrients, vitamins, pharmaceuticals, quasi-drugs, water-soluble drugs applicable to cosmetics other than those described above, antioxidants, buffering agents, antioxidation aids, injection agents; organic powders, pigments, dyes, and colors other than those described above; water, acid components, and alkaline components. These optional components can be appropriately blended at the time of production in an oil phase or a water phase. Further, another cell activating agent may be contained or co-used to enhance the effect of the present invention.

The production method for a powder cosmetic of the present invention can be produced by, for example, a conventional wet production method comprising a slurry step.

Specifically, the powder cosmetic of the present embodiment is obtained by the following procedure. A powder component containing the UV wavelength conversion substance or a phosphor substance and an oil component are mixed using a Henschel mixer^{™}, and further pulverized using a pulverizer to obtain a uniform mixture. An equal amount of a volatile dispersion medium such as water is added to the mixture, and the mixture is mixed using a dispersion mixer to obtain a slurry. The slurry is filled in a middle plate, the volatile dispersion medium is suctioned and removed, and then dried to obtain a powder cosmetic. For details of the wet production method, refer to, for example, Japanese Patent Publication No. 57-60004 or Japanese Examined Patent Publication (Kokoku) No. 61-54766.

The production method for a powder cosmetic of the present invention can be produced, for example, by a conventional wet-mixing/droplet-drying process comprising a slurry step. Specifically, the powder cosmetic of the present embodiment can be obtained by the following procedure.

In the production method for a powder cosmetic of the present invention, the powder components containing the UV wavelength conversion substance and the oil content are crushed and/or pulverized and/or dispersed and mixed in a volatile dispersion medium using a Henschel mixer^{™} to obtain a slurry. The slurry is dried using a flash dryer (for example, a spin flash dryer manufactured by APV Nordic Anhiro, a dry mister manufactured by Hosokawa Micron, or a vertical stirring dryer manufactured by Tsukishima Kikai) to obtain a dry powder. The dry powder or a powder obtained by mixing the dry powder and a pearl pigment is filled in a container and solidified by dry molding. For details of the wet-mixing/droplet-drying process, refer to, for example, Japanese Unexamined Patent Publication (Kokai) No. 2007-055990.

The volatile dispersion medium used in the production method of the present invention is not particularly limited as long as it is a volatile dispersion medium used in conventional wet molding methods, and examples thereof include water, ethyl alcohol, acetone, isopropyl alcohol, and mixtures thereof. Water is particularly preferable.

One aspect of the present invention is a powder cosmetic produced by the production method of the present invention. The powder cosmetic containing the UV wavelength conversion substance produced by the production method including a slurry step has an excellent function as a UV wavelength conversion substance and exhibits a high cell activation effect as compared to powder cosmetics produced by conventional dry production processes.

One aspect of the present invention is a wet powder cosmetic comprising the UV wavelength conversion substance.

In the present description, the phrase "wet powder cosmetic" refers to a powder cosmetic produced by a production method comprising a slurry step, and particularly preferably, refers to a powder cosmetic produced by a "wet production process" which imparts a soft, moist, light, and airy impression.

One aspect of the present invention is a wet powder cosmetic comprising one or more phosphor substances selected from: phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium.

The content of the phosphor substance which can be contained in the wet powder cosmetic of the present invention, relative to the total of the powder cosmetic, is 0.01% by weight or more, 0.1% by weight or more, preferably 0.5% by mass or more, or more preferably 1.0% by weight or more, and 20% by weight or less, preferably 15% by weight or less, more preferably 10% by weight or less, or even more preferably 5% by weight or less. The content relative to the total of the powder cosmetic is 0.01 to 99.99% by weight, 0.01 to 20% by weight, 0.1 to 99.9% by weight, 0.1 to 50% by weight, 0.1 to 40% by weight, 0.1 to 30% by weight, 0.1 to 20% by weight, 0.1 to 10% by weight, or 1 to 10% by weight.

As one aspect of the wet powder cosmetic of the present invention, the phosphor substance of the wet powder cosmetic contains a zinc oxide phosphor. In the powder cosmetic of the present invention, preferably, the content of a zinc oxide phosphor relative to the total of the powder cosmetic is 0.01% by weight or more, 0.1% by weight or more, preferably 0.5% by mass or more, or more preferably 1.0% by weight or more, and 20% by weight or less, preferably 15% by weight or less, more preferably 10% by weight or less, or even more preferably 5% by weight or less. The content relative to the total of the powder cosmetic is 0.01 to 99.99% by weight, 0.01 to 20% by weight, 0.1 to 99.9% by weight, 0.1 to 50% by weight, 0.1 to 40% by weight, 0.1 to 30% by weight, 0.1 to 20% by weight, 0.1 to 10% by weight, or 1 to 10% by weight.

As one aspect of the wet powder cosmetic of the present invention, the phosphor substance of the wet powder cosmetic contains a magnesium titanate phosphor. In the powder cosmetic of the present invention, the content of a magnesium titanate phosphor relative to the total of the powder cosmetic is 0.01% by weight or more, 0.1% by weight or more, preferably 0.5% by mass or more, or more preferably 1.0% by weight or more, and 20% by weight or less, preferably 15% by weight or less, more preferably 10% by weight or less, or even more preferably 5% by weight or less. The content relative to the total of the powder cosmetic is 0.01 to 99.99% by weight, 0.01 to 20% by weight, 0.1 to 99.9% by weight, 0.1 to 50% by weight, 0.1 to 40% by weight, 0.1 to 30% by weight, 0.1 to 20% by weight, 0.1 to 10% by weight, 0.5 to 10% by weight, or 1 to 10% by weight.

As one aspect of the wet powder cosmetic of the present invention, the phosphor substance of the wet powder cosmetic contains phycocyanin. In the powder cosmetic of the present invention, preferably, the content of a phycocyanin relative to the total of the powder cosmetic is 0.00001 % by weight or more, preferably 0.0001% by mass or more, or even more preferably 0.01% by weight or more, and 20% by weight or less, preferably 15% by weight or less, more preferably 10% by weight or less, or even more preferably 5% by weight or less. The content relative to the total of the powder cosmetic is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.01 to 5% by weight, 0.05 to 5% by weight, 0.1 to 5% by weight, 0.1 to 3% by weight, 0.1 to 1% by weight, or 0.01 to 20% by weight.

As one aspect of the wet powder cosmetic of the present invention, the phosphor substance of the wet powder cosmetic contains vitamin B2. In the powder cosmetic of the present invention, preferably, the content of vitamin B2 relative to the total of the powder cosmetic is 0.00001 % by weight or more, preferably 0.0001% by weight or more, and 20% by weight or less, preferably 15% by weight or less, more preferably 10% by weight or less, or even more preferably 5% by weight or less. The content relative to the total of the powder cosmetic is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.005 to 5% by weight, 0.01 to 5% by weight, 0.01 to 1% by weight, 0.01 to 0.5% by weight, or 0.01 to 0.1% by weight.

The powder cosmetic of the present invention can suitably be used in powder cosmetics such as foundation, eyeshadow, cheek color, body powder, perfume powder, baby powder, pressed powder, deodorant powder, loose powder, face powder. The powdered cosmetic of the present invention can be expected to bring about the effects such as prevention of or improvement from wrinkles, spots, skin aging, and photoaging by activating skin cells. Since the powdered cosmetic of the present invention is wet, it feels softer and moister than dry type cosmetics, and when applied to the skin, imparts a transparent finish, giving a soft and airy impression, and has excellent impact resistance.

### EXAMPLES

The present invention will be described in more detail with reference to the following Examples. Note that the present invention is not limited thereto.

### Example 1

### Experiment 1: Cell activation effects of various UV wavelength conversion substances Experiment 1-1: Preparation of UV wavelength conversion substance

A UV wavelength conversion substance was prepared as follows:

### (1) B-phycoerythrin

B-phycoerythrin was obtained from *Porphiridium Cruentum* extract. The absorption spectrum thereof had a peak wavelength of 305 nm, and the emission spectrum thereof had peak wavelengths of 570 nm and 610 nm.

### (2) C-phycocyanin

C-phycocyanin was obtained from *Spirulina platensis* extract. The absorption spectrum thereof had a peak wavelength of 350 nm, and the emission spectrum thereof had peak wavelengths of 640 nm and 700 nm. Linablue by DIC Corp. was used.

### (3) Zinc oxide phosphor

Lumate G by Sakai Chemical Industry Co., Ltd. was used. Lumate G is a zinc oxide phosphor of ZnO doped with a sulfur-containing compound, as described in WO 2018/004006. The absorption spectrum thereof had a peak wavelength of 365 nm, and the emission spectrum thereof had a peak wavelength of 510 nm. (4) Magnesium titanate phosphor

Lumate R by Sakai Chemical Industry Co., Ltd. was used. Lumate R is a magnesium titanate phosphor of MgTiO₃ doped with manganese. The absorption spectrum thereof had a peak wavelength of 365 nm, and the emission spectrum thereof had a peak wavelength within a zone of 660 to 680 nm.

The UV wavelength conversion substances (1) and (2) were dissolved in water to prepare solutions having a concentration of 1% or 5%.

The UV wavelength conversion substances (3) and (4) were dispersed in alcohol to prepare 5% and 10% dispersions.

### Experiment 1-2: Preparation of cell sample

A cell sample was prepared as follows:
1. Human dermal fibroblasts and human skin keratinocytes purchased from Kurabo Industries Ltd. were used. A cell suspension (1 mL) stored in liquid nitrogen was thawed in a water bath (37°C) to an extent that small ice pellets remained, and then diluted with 9 mL of warm medium.
2. The diluted suspension was mixed gently and then transferred to a T75 flask and incubated overnight at 37 °C.
3. The next day, the medium was replaced with 10 mL of fresh medium.
4. The medium was replaced periodically (once every 2 days for fibroblasts and once every 2 to 3 days for keratinocytes) and cell proliferation was continued. Meanwhile, the cells were observed using a microscope, and it was confirmed that the cells grew in the correct form.
5. After the cells reached about 80% confluence, the cells were passaged. The cells were passaged by washing the cells once with 10 mL of warm PBS, adding 5 mL of warm trypsin to a T75 flask, covering the bottom of the flask with a trypsin solution, followed by aspirating at room temperature for 1 minute.
6. The flask was allowed to stand in an oven at 37 °C for (maximum) 2 minutes for fibroblasts and (maximum) 7 minutes for keratinocytes. The cells were observed using a microscope and confirmed to be small and oval.
7. Thereafter, the side of the T75 flask was lightly tapped to release the cells. The cells were observed using a microscope and confirmed to be moving freely.
8. Fibroblasts were resuspended in 5 mL warm FGM (containing 10% serum) and transferred to a sterile 50 mL Falcon tube. An additional 5 mL of warm FGM was used to flush the flask and added to the Falcon tube to ensure transfer of all cells.
9. The cells were centrifuged at 10,000 rpm for 5 minutes (4 °C) and the supernatant was removed taking care not to disturb the pellet of cells.
10. Depending on the cell type, fibroblasts were resuspended in FGM or KGM at a concentration of 2 × 10⁴ cells/well (500 µL), and keratinocytes were resuspended in FGM or KGM at a concentration of 4 × 10⁴ cells/well (500 µL) and plated 24-well plates.
11. Cells were seeded in 24-well plates and the media were changed periodically (once every 2 days for fibroblasts and once every 2 to 3 days for keratinocytes) and the cells were grown until 60 to 70% confluence (depending on the type of experiment) was reached. (Note: Fibroblasts should reach the desired confluency in 24 hours at a cell density of 2 × 10⁴ cells/well. When the cell density is low, for example, 1 × 10⁴ cells/well, it takes 48 hours for fibroblasts to reach the desired confluency.)
12. 24 hours before irradiation, the medium was changed to a supplement-free medium (in the case of keratinocytes) or a medium containing a low concentration of serum (0.5% FCS) (in the case of fibroblasts).

### Experiment 1-3: Ultraviolet irradiation

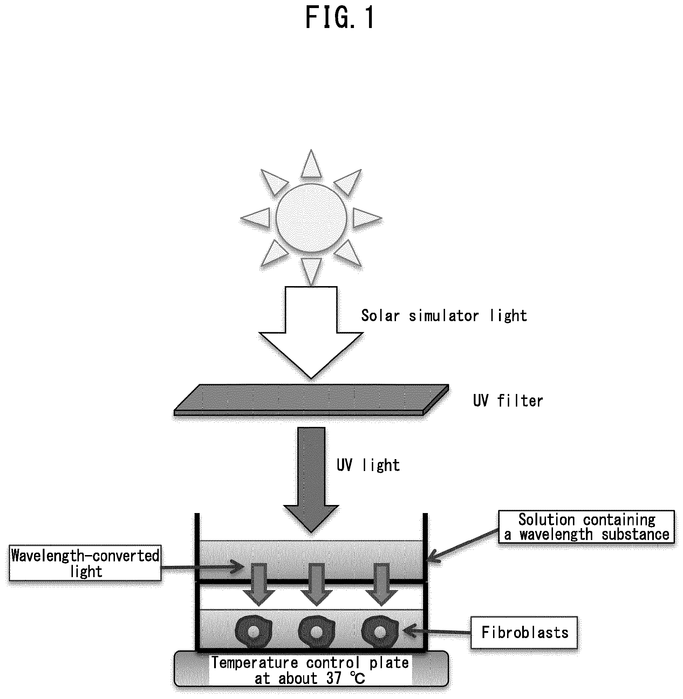

1. A solar simulator was turned on at least 30 minutes before irradiation to warm up a lamp. The solar simulator was set to use a UG11 filter. UG11 filters are filters that allow only UVB to pass and cut light of other wavelengths. The UV light passed through the UG11 filter had a peak wavelength of 300 nm to 385 nm.
2. A temperature control plate was turned on and set to 33 °C.
3. The cells prepared in Experiment 1-2 were washed once with warm PBS.
4. To each well was added 0.5 mL of a warmed Martinez solution (145 mM NaCl, 5.5 mM KCl, 1.2 mM MgCl₂·6H₂O, 1.2 mM NaH₂PO₄·2H₂O, 7.5 mM HEPES, 1 mM CaCl₂, and 10 mM D-glucose).
5. As depicted in FIG. 1, cell wells were placed on a plate. Then, 0.4 ml of a solution containing the UV wavelength conversion substances (1) to (4) prepared in Experiment 1-1 was injected into each well of a 24-well plate. The wells containing cells were placed to be covered, such that the cell solution was irradiated with UV light through the solution of the UV wavelength conversion substances without direct contact between the UV wavelength conversion substance solution and the cell solution.
6. Irradiation was carried out to a total dose of 100 mJ/cm². Further, as controls, there were prepared a sample of cells irradiated directly with UV light without placing a plate of a UV wavelength conversion substance on cell wells and a sample of cells cultured in the dark without irradiation with UV light.
7. After irradiation, the Martinez was replaced with warm KGM (without supplements) or FGM (containing 0.5% FCS) and the plate was returned to the incubator at 37 °C.

### Experiment 1-4: Measurement of cell activity

After the completion of Experiment 1-3, the cells maintained in the incubator for 48 hours were used to measure the activity by the following method:
1. A medium (KGM medium without supplements or 0.5% FCS-containing FGM medium) was supplemented with 10% AlamarBlue and warmed to 37 °C. (Keep the solution in the dark.)
2. The medium in the wells was replaced with 500 µL of the above 10% AlamarBlue solution, and the plate was returned to the incubator at 37 °C for about 3 hours. Control wells were also maintained in the incubator. These solutions were maintained in the dark to protect them from light.
3. After 3 hours, 100 µL aliquots were collected and transferred to a black 96-well plate.
4. Fluorescence measurement values at 544 nm/590 nm were read using a fluorometer (OPwave +, Ocean Photonics).

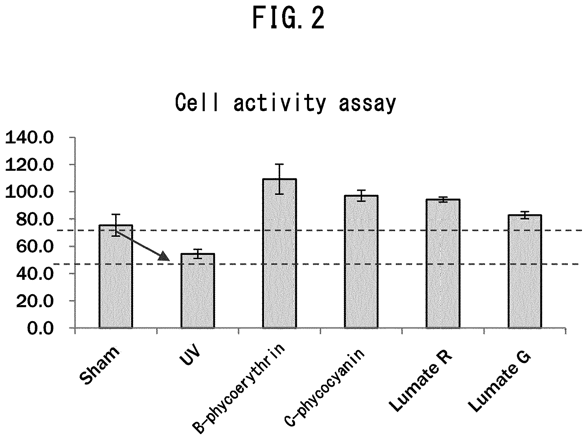

The results are shown in FIG. 2. UV irradiation reduced cell activity compared to the control without irradiation. However, the activity of cells irradiated with UV through any UV wavelength conversion substance was increased compared to the control without irradiation. From the above results, it was found that although the cell activity was decreased by UV irradiation, the decrease in cell activity is suppressed using a UV wavelength conversion substance.

### Example 2: Influence of concentration of UV wavelength conversion substance or intensity of UV on cell activity

The same method as in Experiment 1 was employed except that C-phycocyanin was used as the UV wavelength conversion substance, a cell culture was covered with a plate of a solution containing C-phycocyanin at 0%, 0.4%, or 2%, and irradiated with UV at a dose of 0, 10, 25, 50, 75, or 100 mJ/cm² .

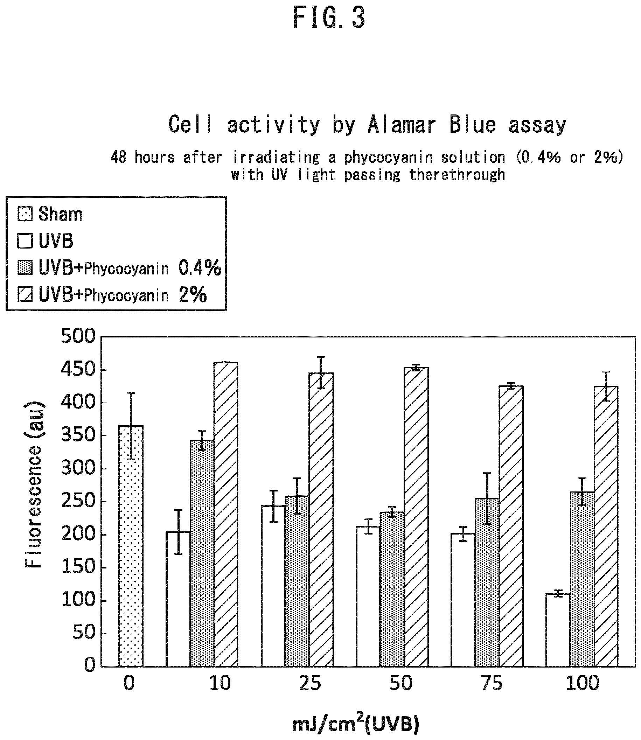

The results are shown in FIG. 3. When the UV wavelength conversion substance was not used, the cell activity decreased as the UV irradiation amount increased. However, when 0.4% C-phycocyanin was added, the decrease in cell activity was suppressed even when UV irradiation was carried out, and when 2% C-phycocyanin was added, the cell activity was even increased compared to the case where UV irradiation was not carried out. From the above results, it was found that although the cell activity was decreased by UV irradiation, the use of a UV wavelength conversion substance not only suppressed the decrease in cell activity in a concentration-dependent manner, but also enhanced the cell activity.

### Example 3: Restoration of cell activity decreased by UV irradiation

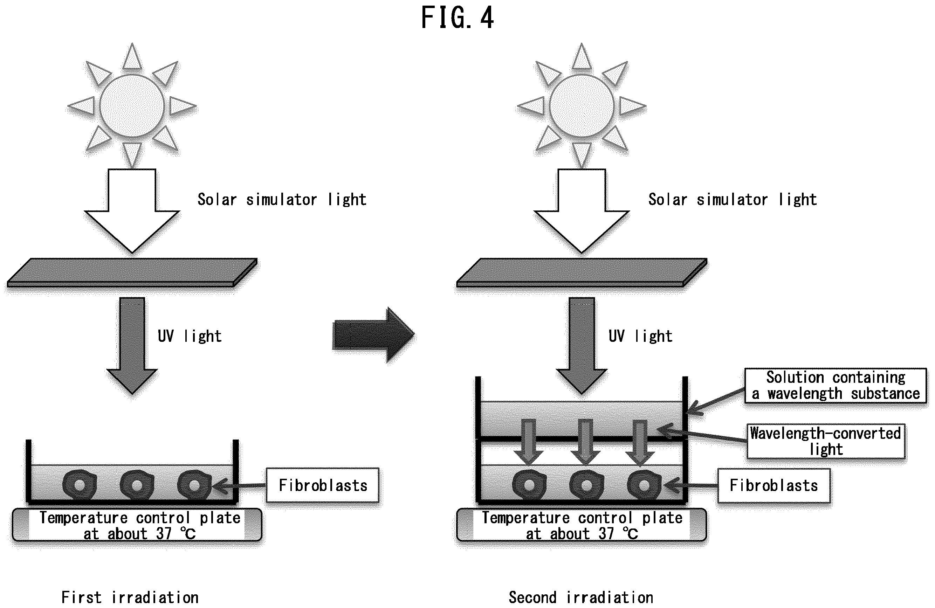

The same method as in Experiment 1 was employed except that as shown in FIG. 4, UV irradiation was carried out without using a UV wavelength conversion substance until the irradiation amount reached 400 mJ/cm² to once decrease the cell activity, and a cell culture was covered with a plate of a solution containing C-phycocyanin as the UV wavelength conversion substance at 0%, 0.4%, or 2%, and irradiated with UV at a dose of 0, 10, 25, 50, 75, 100, or 200 mJ/cm ² .

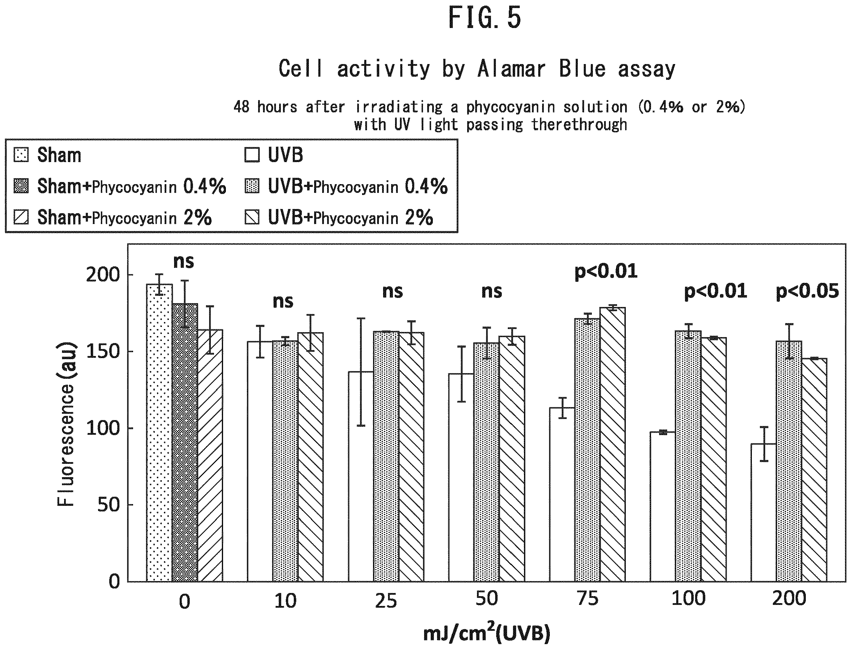

The results are shown in FIG. 5. It can be seen that cell activity was recovered by subjecting even cells having once decreased activity by UV irradiation without using a UV wavelength conversion substance to UV irradiation using a UV wavelength conversion substance. This effect of C-phycocyanin was equivalent at concentrations of 0.4% and 2%, suggesting a sufficient cell activation effect at 0.4%. On the other hand, when UV irradiation was carried out without using a UV wavelength conversion substance, the cell activity decreased in a UV dose-dependent manner.

The results for human dermal fibroblasts are described above. Similar results were also observed for keratinocytes (data not shown). From these results, it was found that a UV wavelength conversion substance not only suppressed a decrease in cell activity due to UV irradiation, but also has an effect of activating cells using UV light. When skin cells are activated, prevention of and improvement from wrinkles, spots, skin aging, photoaging, etc. are expected.

From Examples 1 to 3 above, it was considered that a UV wavelength conversion substance converts the wavelength of ultraviolet, and emitted visible light (fluorescence having a main wavelength of 500 nm to 700 nm) activates skin cells such as fibroblasts and corneocytes. Thus, various powder cosmetics containing a UV wavelength conversion substance were produced, and the amount of fluorescence emitted during ultraviolet irradiation was evaluated.

For fluorescence measurement, the powder cosmetic was applied to an S plate (refer to Japanese Registered Patent Publication No. 4453995) at 2 mg/cm² and dried to prepare a coating film of the powdered cosmetic. The obtained coating film was irradiated with ultraviolet having a wavelength of 365 nm, and a fluorescence integrated value in a wavelength region of 400 to 600 nm was measured using a fluorescence spectrophotometer RF-5300PC (Shimadzu Corporation).

### Example 4: Effect of UV wavelength conversion function of medicament

The powdered cosmetics (Formulation Examples 0 and 1) having the compositions shown in Table 1 were produced according to a conventional wet production process. Water was used as the volatile dispersion medium. As Comparative Examples, identical compositions were produced by a dry production process (compression molding). As the UV wavelength conversion substance, a stearic acid-treated zinc oxide phosphor was used (Formulation Example 1). Coating films of the obtained powder cosmetics were irradiated with ultraviolet having a wavelength of 365 nm, and the fluorescence integrated value at a wavelength of 400 to 600 nm was measured. Though Formulation Example 1 had the same composition, the powder cosmetic produced by a wet production process had a higher fluorescence value than the powder cosmetic produced by the dry production process (100% for the dry production process and 107% for the wet method). In Formulation Example 0, which contained no zinc oxide phosphor, no fluorescence value was observed regardless of whether the dry process or the wet production process was used.

From the foregoing, it was found that the powder cosmetic by the wet production process was a formulation form suitable for exhibiting the function of the UV wavelength conversion substance.

**[Table 1]**

| Formulation composition | Formulation Example 0 | Formulation Example 1 |
|---|---|---|
| Elastomer-treated talc | balance | balance |
| Synthetic gold mica iron | 10 | 10 |
| Mica | 10 | 10 |
| Boron nitride | 10 | 10 |
| Fine particulate titanium oxide | 3 | 3 |
| (Vinyl dimethicone/methicone silsesquioxane) crosspolymer | 15 | 15 |
| Nylon powder | 5 | 5 |
| Fine particulate zinc oxide | 5 | |
| Zinc oxide phosphor | | 5 |
| Pigment-grade titanium oxide | 10 | 10 |
| Red iron oxide | 0.15 | 0.15 |
| Yellow iron oxide | 1.5 | 1.5 |
| Black iron oxide | 0.1 | 0.1 |
| Titanated mica | 1 | 1 |
| Barium sulfate | 1 | 1 |
| Preservative | q.s. | q.s. |
| Dimethicone | 2 | 2 |
| Caprylyl methicone | 2 | 2 |
| Glyceryl tri(caprylate/caprate) | 3 | 3 |
| Ethylhexyl methoxycinnamate | 5 | 5 |

The embodiments of the production method of the present invention and the powder cosmetic according to the production method are described above. However, the present invention is not limited thereto, and can be modified as appropriate without departing from the spirit of the invention.

## Claims

1. A production method of a powder cosmetic containing a UV wavelength conversion substance, comprising a slurry step.

2. The production method according to claim 1, which is a wet production process.

3. The production method according to claim 1, which is a wet-mixing/droplet-drying process.

4. The production method according to any one of claims 1 to 3, wherein the UV wavelength conversion substance comprises one or more UV wavelength conversion substances selected from: phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium.

5. The production method according to any one of claims 1 to 4, wherein the content of the UV wavelength conversion substance is 0.01 to 20% by weight relative to the total of the powder cosmetic.

6. A production method of a powder cosmetic containing one or more phosphor substances selected from: phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, lycopene, salicylic acid, capsicum extract, capsicum color, paprika color, gardenia color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a magnesium titanate phosphor; and a calcium phosphate phosphor of a calcium phosphate doped with cerium, the method comprising a slurry step.

7. The production method according to claim 6, wherein the content of the phosphor substance is 0.01 to 20% by weight relative to the total of the powder cosmetic.

8. The production method according to any one of claims 1 to 7, wherein the powder cosmetic is a foundation, eyeshadow, cheek color, body powder, perfume powder, baby powder, pressed powder, deodorant powder, loose powder, or face powder.

9. The production method according to any one of claims 1 to 8, wherein a fluorescence intensity increasing effect is exhibited.

10. The production method according to any one of claims 1 to 9, wherein a cell activation effect is exhibited.

11. A powder cosmetic produced by the production method according to any one of claims 1 to 10.

12. A wet powder cosmetic comprising a UV wavelength conversion substance.

13. The wet powder cosmetic according to claim 12, wherein the UV wavelength conversion substance comprises one or more UV wavelength conversion substances selected from:
phycocyanin, phycoerythrocyanin, phycoerythrin, vitamin A, β-carotene, vitamin K, vitamin B1,
vitamin B2, vitamin B2 derivatives, vitamin B6, vitamin B12, folic acid, niacin, lycopene,
salicylic acid, capsicum extract, capsicum color, paprika color, gardenia color, safflower color,
turmeric color, cochineal color, perilla color, red cabbage color, flavonoid, carotenoid, quinoid, porphyrins, anthocyanins, polyphenols, Red No. 401, Red No. 227, Red No. 504, Red No. 218,
Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401,
Herindon pink, Yellow No. 401, Benzidine yellow G, Blue No. 404, Red No. 104,
meta-aminophenol; a blue phosphor comprising an amorphous silica particle, cerium, and
phosphorus and/or magnesium; a red phosphor comprising a europium-activated compound of a mixed crystal of an alkaline earth metal sulfide and a gallium compound; a zinc oxide phosphor,
a magnesium titanate phosphor; and a calcium phosphate phosphor of a calcium phosphate doped with cerium.

14. The wet powder cosmetic according to claim 12 or 13, wherein the content of the UV wavelength conversion substance is 0.01 to 20% by weight relative to the total of the cosmetic.

15. A wet powder cosmetic comprising one or more phosphor substances selected from:
phycocyanin, phycoerythrocyanin, phycoerythrin, vitamin A, β-carotene, vitamin K, vitamin B1,
vitamin B2, vitamin B2 derivatives, vitamin B6, vitamin B12, folic acid, niacin, lycopene,
salicylic acid, capsicum extract, capsicum color, paprika color, gardenia color, safflower color,
turmeric color, cochineal color, perilla color, red cabbage color, flavonoid, carotenoid, quinoid, porphyrins, anthocyanins, polyphenols, Red No. 401, Red No. 227, Red No. 504, Red No. 218,
Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401,
Herindon pink, Yellow No. 401, Benzidine yellow G, Blue No. 404, Red No. 104,
meta-aminophenol; a blue phosphor comprising an amorphous silica particle, cerium, and
phosphorus and/or magnesium; a red phosphor comprising a europium-activated compound of a mixed crystal of an alkaline earth metal sulfide and a gallium compound; a zinc oxide phosphor,
a magnesium titanate phosphor; and a calcium phosphate phosphor of a calcium phosphate doped with cerium.

16. The wet powder cosmetic according to claim 15, wherein the content of the phosphor substance is 0.01 to 20% by weight relative to the total of the powder cosmetic.

17. The wet powder cosmetic according to any one of claims 12 to 16, which is a foundation, eyeshadow, cheek color, body powder, perfume powder, baby powder, pressed powder, deodorant powder, loose powder, or face powder.

18. The wet powder cosmetic according to any one of claims 12 to 17, which exhibits a fluorescence intensity increasing effect.

19. The wet powder cosmetic according to any one of claims 12 to 18, which exhibits a cell activation effect.
